# EUROPEAN PATENT SPECIFICATION

(11) **EP 2 082 690 B1**
(45) Date of publication and mention of the grant of the patent: **20.06.2012**
(21) Application number: 08100878.1
(22) Date of filing: 24.01.2008
(51) Int. Cl.: A61F 2/24, A61B 17/00

(54) **Medical device to assist diastolic function and prevent ventricular enlargement**
Medizinische Vorrichtung zur Unterstützung der diastolischen Funktion und zur Verhinderung einer Herzkammervergrößerung
Dispositif médical pour assister la fonction diastolique et éviter l'élargissement ventriculaire

(43) Date of publication of application: 29.07.2009
(73) Proprietor: Kardium, Inc., Richmond, BC V6V 2K5 (CA)
(72) Inventor: Gelbart, Daniel, Vancouver British Columbia V6T 1B4 (CA); Lichtenstein, Samuel Victor, Vancouver British Columbia V6N 1J1 (CA)
(74) Representative: Moore, Barry

(56) References cited:
- WO-A-2005/102181
- US-A1- 2003 050 685
- US-A1- 2004 002 626
- US-A1- 2004 243 170
- US-A1- 2006 229 491
- US-A1- 2006 293 698

## Description

### BACKGROUND

### Technical Field

This disclosure is related to percutaneously implanted medical devices suitable for treating heart failure such as congestive heart failure and diastolic dysfunction as disclosed in the appended claims.

### Description of the Related Art

Diastolic dysfunction (*i.e*., insufficient expansion of the left ventricle during the diastolic phase) and general deterioration of the left ventricular performance are very common problems, affecting about 5 million people in the U.S. alone. The problems can be triggered by a myocardial infarction or develop slowly over time. More background data on congestive heart failure can be found on the internet at:
http://healthlink.mcw.edu/article/928348606.html and many other medical sources.

Treatment of diastolic dysfunction and general deterioration of the left ventricle can be classified generally into three methods: surgery to change the shape of the left ventricle, wrapping the heart in an elastic net, or introducing reinforcing structures via a catheter into the left ventricle. The first two methods require extensive surgery. Minimally invasive or percutaneous procedures such as those disclosed by U.S. Patent Application Publication Nos. 2005/0015109; 2004/0243170; 2004/0249408 and 2006/0025800 address the need of strengthening the heart wall to resist remodeling and enlargement due to systolic pressure, but do not improve diastolic expansion to allow better filling of the left ventricle with blood. In many cases previous approaches actually sacrifice diastolic function in exchange for preventing the abnormal enlargement of the left ventricle that often follows myocardial infarction. For example, wrapping the heart in an elastic net will assist systolic action and will limit left ventricle enlargement, but will interfere with diastolic function as it will require more force to expand the left ventricle and stretch the net. The same is true of any rigid internal reinforcement.

Other minimally invasive or percutaneous procedures such as the one disclosed by U.S. Patent Application Publication No. 2007/0161846 assist diastolic function to some degree, but fail to adequately resist remodeling and enlargement of the ventricle. The device disclosed by U.S. Patent Application Publication No. 2007/0161846 is secured to a ventricle at a peripheral edge and does not bond to the ventricle through the formation of scar tissue. Rather, the device typically includes a stand-off that spaces the ribs of the device a distance away from the ventricle wall, thus providing little structural support. Furthermore, the device does not include a number of restraining members to limit radial expansion of the device. In fact, Application Publication No. 2007/0161846 teaches away from the use of restraining members by disclosing the use of a strand extending around the periphery of the device to create an outward expansive force as opposed to a restraining force. As such, the device fails to provide structural support to the ventricle wall sufficient to prevent ventricular enlargement.

US 2003050685 (A1) describes a method and a device for improving cardiac function. The device is packaged in a collapsed state in an end of a catheter. Portions of a frame construction of the device spring outwardly when the catheter is withdrawn from the device. Anchoring formations on the frame construction secure the frame construction to a myocardium of the heart. A membrane secured to the frame construction then forms a division between volumes of an endocardial cavity of the heart on opposing sides of the membrane.

US 2004243170 (A1) describes introducing a collapsed reinforcing element through the skin into the vascular system of the human. The method may include delivering the reinforcing element into a left ventricle through the arteries. Once inside the left ventricle, the reinforcing element may be expanded to an expanded shape. In certain embodiments, a reinforcing element may be used to structurally reinforce a portion of an endocardial surface of a heart. The reinforcing element may include a preshaped patch and/or a plurality of preshaped flexible conduits. The method may include deploying the reinforcing element soon after a myocardial infarction to inhibit naturally occurring remodeling of the heart. The reinforcing element may be deployed with or without the use of a shaper. In some embodiments, a reinforcing element may include an externally positioned apparatus configured to substantially reshape a portion of an interior chamber of a heart.

Improved medical devices are desirable.

### BRIEF SUMMARY

A medical device that assists diastolic function is highly desirable. The device should be implantable percutaneously, via a catheter. The device should also limit the enlargement of the left ventricle, thus solving two major problems of congestive heart failure in a single percutaneous procedure.

Various embodiments of a medical device configured for percutaneous installation in a ventricle as disclosed in claim 1 comprise a plurality of elongated arms that are compressably elastically deformable from a relatively radially expanded configuration to a relatively radially unexpanded configuration. In a relatively radially expanded configuration, the plurality of elongated arms press against or attach to the walls of the ventricle and over time may bond firmly to the walls via scar tissue formation. The elongated arms help the ventricle expand and fill with blood during the diastolic period and preferably have little affect on systolic performance.

The maximum expansion of elongated arms is limited by restraining members thus strengthening the ventricular walls and further limiting the effects of congestive heart failure.

The elastic range of elongated arms is increased by adding bends to the arms, for example, by adding small loops, V-shape bends or other bend configurations that distribute elastic deformation over a greater length of the arms. Such bends are useful for allowing installation via a smaller diameter catheter and reducing material fatigue induced failures.

In other examples not forming part of the invention, a medical device configured for percutaneous installation in a ventricle comprise a plurality of elements that form an elongated mesh tubular structure that is compressably elastically deformable from a relatively radially expanded conical configuration to a relatively radially unexpanded cylindrical configuration. In a relatively radially expanded conical configuration, the elongated mesh tubular structure presses against or attaches to the walls of the ventricle and over time may bond firmly to the walls via scar tissue formation. The elongated mesh tubular structure helps the ventricle expand and fill with blood during the diastolic period and preferably has little affect on systolic performance. The elongated mesh tubular structure strengthens the ventricular walls and further limits the effects of congestive heart failure. In some embodiments, some of the elements forming the elongated mesh tubular structure include bends that increase the elastic range of the structure. Such bends can be of various configurations, including, for example, loops and V-shape bends.

At least one embodiment as disclosed in the appended claims may be summarized as a percutaneously implantable combined diastolic assist and dilation prevention medical device including a plurality of elongated arms that extend from an apex and have a respective distal end spaced from the apex, the elongated arms compressably elastically deformable from a relatively radially expanded configuration to a relatively radially unexpanded configuration, where the respective distal end of each of the elongated arms is spaced relatively away from one another in the relatively radially expanded configuration and the respective distal ends of each of the elongated arms is spaced relatively close to one another in the relatively radially unexpanded configuration, wherein each of the elongated arms has at least one bend therein such that the elongated arm is bent back on itself, the elongated arms elastically biased toward the relatively radially expanded configuration while in the relatively unexpanded configuration, the at least one bend increasing an elastic range of the elongated arm.

The elongated arms of the medical device may include a first bend proximate the apex and a second bend proximate the distal end of the elongated arms. The elongated arms may include at least a third bend between the first and second bends. The at least one bend of the medical device may form a complete loop. The medical device (in an example not forming part of the invention) may further include a number of restraining members, at least one of the restraining members extending between each pair of successively radially adjacent ones of the elongated arms, wherein the restraining members limit a radial expansion of the elongated arms relative to one another. The medical device includes a number of restraining members, at least one of the restraining members extending between each pair of opposing ones of the elongated arms, wherein the restraining members limit a radial expansion of the elongated arms relative to one another. Pairs of opposed ones of the elongated arms may be a unitary elastic member. Pairs of opposed ones of the elongated arms may be an integral member. Pairs of opposed ones of the elongated arms may be distinct members, each physically coupled to a common connector member. The common connector member may include a circular ring wherein the plurality of elongated arms may be connected to the periphery of the circular ring and spaced at substantially equal intervals. The medical device may further include at least one barb located at the common connector member and oriented outwardly from the device. The common connector member may include a threaded member configured to engage corresponding threads of a catheter assembly. The medical device may further include a catheter assembly capable of retaining the elongated arms in the relatively radially unexpanded configuration within a cavity of the catheter assembly and having delivery means adapted to engage the common connector member. The elongated arms may comprise a shape memory material. The elongated arms may comprise Nitinol. At least some of the elongated arms may have at least one barb at least proximate a distal end thereof. At least some of the elongated arms may have a surface finish at least proximate a distal end thereof. At least some of the elongated arms may have a load spreading structure at least proximate a distal end thereof. The medical device in the radially unexpanded configuration may be capable of retention in a catheter assembly. The medical device in the radially expanded configuration may be in the form of a cone-like shape.

At least one example not forming part of the invention may be summarized as a percutaneously implantable combined diastolic assist and dilation prevention medical device, including a plurality of elements that form an elongated mesh tubular structure having a first end and a second end opposed to the first end, the elongated mesh tubular structure compressably elastically deformable from a relatively radially expanded conical configuration with an apex at the first end and the second end having a first diameter to a relatively radially unexpanded cylindrical configuration with the second end having a second diameter smaller than the first diameter, the elongated mesh tubular structure elastically biased toward the relatively radially expanded configuration while in the relatively unexpanded configuration.

The elongated mesh tubular structure may be a woven, knitted or braided mesh tubular structure. The elongated mesh tubular structure may be a wire welded mesh tubular structure. The plurality of elements that form the elongated mesh tubular structure may have at least one bend, the at least one bend increasing an elastic range of the elongated mesh tubular structure. The at least one bend may form a complete loop. The at least one bend may form a V-shape. The bends in the elements having the at least one bend may limit a radial expansion of the elongated mesh tubular structure. The elements forming the elongated mesh tubular structure may comprise unitary elastic members. The elements forming the elongated mesh tubular structure may comprise pieces of a shape memory material. The elements forming the elongated mesh tubular structure may comprise pieces of Nitinol. The medical device may further include at least one barb located at the apex and oriented outwardly from the elongated mesh tubular structure. The medical device may further include a catheter assembly capable of retaining the elongated mesh tubular structure in the relatively radially unexpanded cylindrical configuration within a cavity of the catheter assembly. The medical device may further include a plurality of barbs extending from the elongated mesh tubular structure along a length thereof. The elongated mesh tubular structure may have a rough exterior surface finish.

At least one embodiment as disclosed in the appended claims may be summarized as a combined diastolic assist and dilation prevention medical device configured for percutaneous installation having a plurality of elongated arms that extend from an apex and have a respective distal end spaced from the apex, the elongated arms compressably elastically deformable from a relatively radially expanded configuration to a relatively radially unexpanded configuration, where the respective distal end of each of the elongated arms is spaced relatively away from one another in the relatively radially expanded configuration and the respective distal end of each of the elongated arms is spaced relatively close to one another in the relatively radially unexpanded configuration, the elongated arms elastically biased toward the relatively radially expanded configuration while in the relatively unexpanded configuration; and a number of restraining members, at least one of the restraining members extending between at least a first and a second one of the elongated arms to provide a limit to a radial expansion of the elongated arms relative to one another.

The at least a first and a second one of the elongated arms may be successively radially adjacent ones. The at least a first and a second one of the elongated arms may be opposing ones. The restraining members may be flexible stainless steel cables. Pairs of opposed ones of the elongated arms may be a unitary elastic member. Pairs of opposed ones of the elongated arms may be an integral member. Pairs of opposed ones of the elongated arms may be distinct members, each physically coupled to a common connector member. The common connector member may include a circular ring wherein the plurality of elongated arms may be connected to the periphery of the circular ring and spaced at substantially equal intervals. The medical device may further include at least one barb located at the common connector member and oriented outwardly from the device. The common connector member may include a threaded member, the threaded member configured to engage corresponding threads of a catheter assembly. The medical device may further include a catheter assembly capable of retaining the elongated arms in the relatively radially unexpanded configuration within a cavity of the catheter assembly and having delivery means adapted to engage the common connector member. The elongated arms may comprise a shape memory material. The elongated arms may comprise Nitinol. At least some of the elongated arms may have at least one barb at least proximate a distal end thereof. At least some of the elongated arms may have a surface finish at least proximate a distal end thereof.

Further objects and advantages of the devices and methods taught herein will become clear by studying the disclosure, drawings and claim.

### BRIEF DESCRIPTION OF THE SEVERAL VIEWS OF THE DRAWINGS

In the drawings, identical reference numbers identify similar elements. The sizes and relative positions of elements in the drawings are not necessarily drawn to scale. For example, the shapes of various elements and angles are not drawn to scale, and some of these elements are arbitrarily enlarged and positioned to improve drawing legibility. Further, the particular shapes of the elements as drawn, are not intended to convey any information regarding the actual shape of the particular elements, and have been solely selected for ease of recognition in the drawings.
Figure 1 is a cross-sectional view of a heart showing installation of a medical device using a catheter assembly.
Figure 2-A is a cross-sectional view of a medical device retained in a catheter assembly for percutaneous installation in a left ventricle, according to one illustrated embodiment.
Figure 2-B is an isometric view of a medical device installed in a left ventricle, according to one illustrated embodiment.
Figure 3-A is an isometric view of a medical device for percutaneous installation in a heart in a relatively radially expanded configuration, according to one illustrated embodiment.
Figure 3-B is an isometric view of a medical device for percutaneous installation in a heart in a relatively radially expanded configuration, according to one illustrated embodiment.
Figure 4-A is a plan view showing one configuration of restraining members extending between elongated arms, according to one illustrated embodiment.
Figure 4-B is a plan view showing one configuration of restraining members extending between elongated arms, according to one illustrated embodiment.
Figure 4-C is a plan view showing one configuration of restraining members extending between elongated arms, according to one illustrated embodiment.
Figure 4-D is a plan view showing one configuration of restraining members extending between elongated arms, according to one illustrated embodiment.
Figure 5 is an isometric view of a medical device being retrieved from a left ventricle, according to one illustrated embodiment.
Figure 6-A is an isometric view of an elongated mesh tubular structure retained in a catheter assembly, according to one illustrated embodiment.
Figure 6-B is an isometric view of an elongated mesh tubular structure in a relatively radially unexpanded cylindrical configuration, according to one illustrated embodiment.
Figure 6-C is an isometric view of an elongated mesh tubular structure in a relatively radially expanded conical configuration, according to one illustrated embodiment.
Figure 7-A is a front view of an elastically deformable element having at least one bend, according to one example which does not form part of the invention.
Figure 7-B is a front view of an elastically deformable element having at least one bend, according to one example which does not form part of the invention.
Figure 7-C is a front view of an elastically deformable element having at least one bend, according to one example which does not form part of the invention.
Figure 8-A is a partial view of an elastically deformable element of a medical device comprising a barb and a load spreading structure, according to one example which does not form part of the invention.
Figure 8-B is a partial view of an elastically deformable element of a medical device comprising a barb and a load spreading structure, according to one example which does not form part of the invention.
Figure 8-C is a partial view of an elastically deformable element of a medical device comprising a surface finish, according to one example which does not form part of the invention.
Figure 8-D is a partial view of a medical device comprising a plurality of elastically deformable elements, barbs and a membrane structure, according to one example which does not form part of the invention.

### DETAILED DESCRIPTION

With reference to the attached drawings, a medical device 4 for percutaneous installation comprises a plurality of elastically deformable elements 6, such as elongated arms 6a and elements 6b forming elongated mesh tubular structure 7, to assist diastolic function by gently trying to expand the left ventricle 2 of a heart 3. The elastic force is preferably a small fraction of the force during systolic contraction, thus the device 4 preferably has little affect on the systolic pressure or ejected volume. It is well known that diastolic dysfunction is a major cause of cardiovascular failure, as it is far more common than systolic dysfunction. After some time (weeks to months) scar tissue may permanently bind the plurality of elastically deformable elements 6 to the ventricular wall 19. At this point the device 4 also prevents ventricular enlargement, acting as reinforcement to the ventricular wall 19 and limiting the maximum size of the left ventricle 2. Since the enlargement of the left ventricle as a result of congestive heart failure or infarct is gradual, scar tissue will have a chance to form before full bond strength is needed between the device 4 and ventricular wall 19.

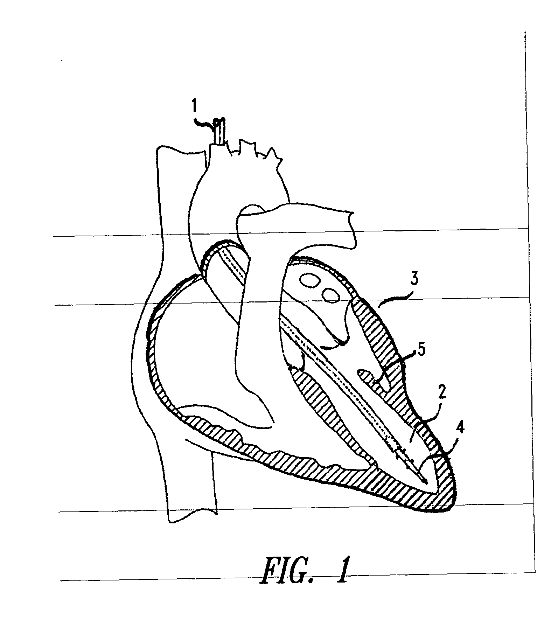

Figure 1 shows an embodiment of a medical device 4 installed via a catheter assembly 1 inserted through the aorta into the left ventricle 2 of a heart 3 having papillary muscles 5. A catheter of the catheter assembly 1 may be of any size; however, the catheter size is preferably in the same range as other percutaneous cardiac procedures, using sizes in the range of about 12 Fr to 28 Fr (about 4 to 9mm). While Figure 1 illustrates the use of a catheter assembly 1 to enter the left ventricle 2 via the mitral valve, it is appreciated that device 4 is capable of installation by various alternate methods. For example, device 4 can be installed in the left ventricle 2 also via the aortic valve, by piercing the apex of the left ventricle or by an incision at any convenient point. Although percutaneous installation is the preferred method, the device can be installed during conventional cardiac surgery.

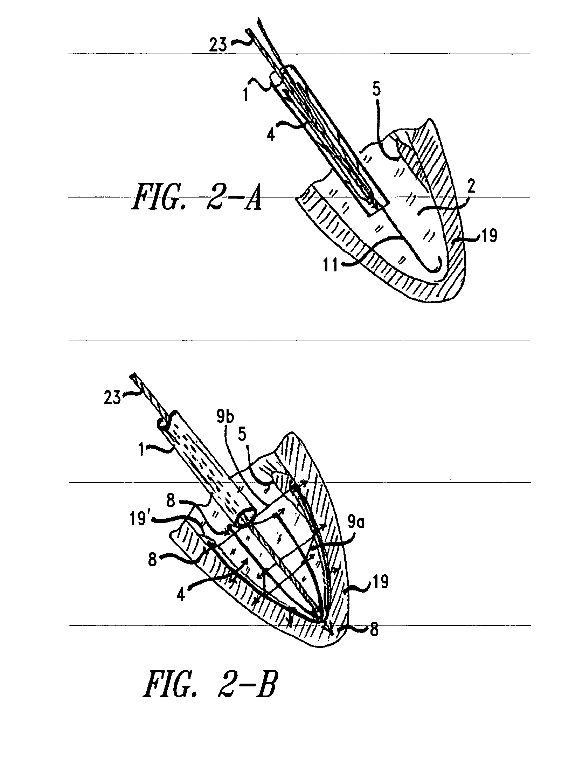

Figure 2-A illustrates an embodiment of a medical device 4 inside catheter assembly 1. In some embodiments, device 4 is held by flexible cable 23, which may be used to push device 4 through the catheter assembly 1, typically via a hemostatic seal outside the body (not shown). A guide wire 11 may be used to guide the catheter assembly 1 into the left ventricle 2.

Figure 2-B illustrates an embodiment of a medical device 4 after installation in the left ventricle 2. Device 4 expands to a relatively radially expanded configuration in the left ventricle 2. Ventricular contractions may help embed optional barbs 8 into ventricular wall 19. Over time, scar tissue 19' may form a bond between device 4 and wall 19.

The maximum expansion of device 4 is limited not only by the ventricular wall 19 but by restraining members 9. Restraining members 9 are connected between opposing arms rather than between adjacent arms to allow the restraining members 9 to clear the papillary muscles 5, allowing the device 4 to cover a larger part of the left ventricle 2. As shown in Figure 2-B, the papillary muscles 5 may fit between two restraining members 9b of device 4.

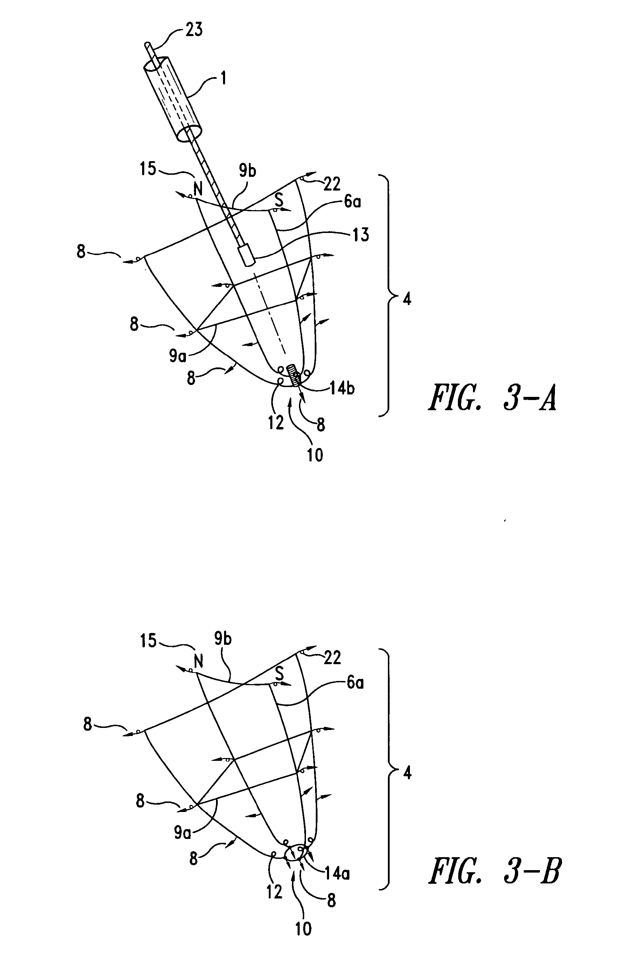

Figures 3-A and 3-B show embodiments of a medical device 4 in a relatively radially expanded configuration. The device 4 has a plurality of elastically deformable elements in the form of elongated arms 6a. In some embodiments, the elongated arms 6a are equipped with barbs 8 and restraining members 9a, 9b (collectively 9). The arms 6a can be made from any durable, elastically deformable material, for example, Nitinol, spring tempered stainless steel, plated beryllium copper or polymeric material.

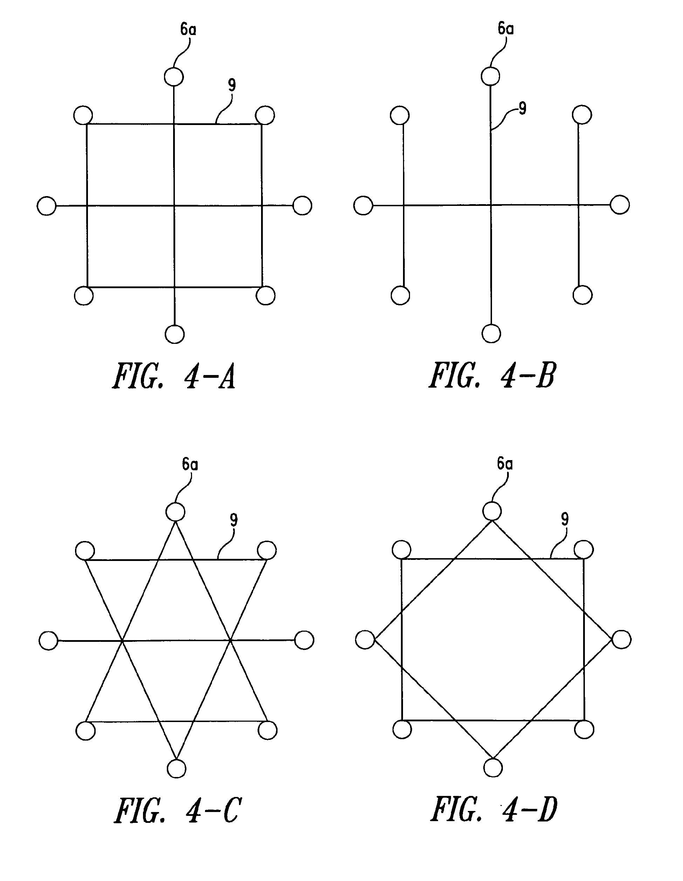

Restraining members 9 can be flexible steel cables, polymeric cables, flexible ribbons or similar flexible members. The purpose of restraining members 9 is to limit the maximum dilation of the left ventricle 2 and prevent ventricular enlargement (after elongated arms 6a bond to ventricle wall 19 by scar tissue 19'). The number of restraining members 9 of device 4 can vary, for example the preferred embodiment may have from 3 to 12 restraining members 9. Restraining members 9 can connect any combination of elongated arms, for example not forming part of the invention, adjacent elongated arms, such as restraining members 9a, or opposing elongated arms as disclosed in the appended claims, such as restraining members 9b. Various configurations of restraining members 9 representing only a small fraction of possible configurations are illustrated in Figures 4-A to 4-B.

In some embodiments, a common connector member 14 is located at the apex 10 of the device 4, such as is illustrated in Figure 3-A. In some embodiments, the common connector member 14 may be coupled to or formed integrally with a threaded member 14b for temporary attachment to flexible cable 23 of catheter assembly 1 via corresponding threads 13, as illustrated in Figure 3-A. In other embodiments, the common connector member 14 comprises a ring 14a wherein elongated arms 6a are attached to its periphery by conventional attachment means, for example, by soldering or welding, as illustrated in Figure 3-B.

The force that the elongated arms 6a exert on ventricle wall 2 is approximated by the equation F=k(x+a), where k represents the spring constant, a represents the preload (amount of spring preload beyond the fully dilated position) and x represents the ventricular wall movement. The spring constant, k, is preferably selected so as to not interfere with systolic function while aiding diastolic filling. By way of example, the total force the ventricular wall is capable of exerting on each one of the elongated arms 6a is about 20-30N (about 2-3 Kg) and the average movement during contraction is about 1-2cm. In order to limit the effect on systolic operation the total force is preferably below about 10% of systolic force, or about 2N. If a preload of 2cm is chosen, the spring constant can be calculated from the equation: 2N=k(0.02m + 0.02m), k=50N/m. The size of wire or other elastically deformable material forming elongated arms 6a is determined by k and is typically in the range of 0.5-1 mm. If desired, a non linear spring can be used or a nearly constant force spring can be used. A constant force spring is approximated by using a very low k spring with a very large preload, since F=k(x+a), the same force F can be achieved by a very low spring constant k and large preload a, thus most of the force comes from the term ka which is constant.

In accordance with some embodiments, it is beneficial to know the orientation of the device 4 inside the left ventricle 2 in order to place device 4 correctly relative to the papillary muscles. This can be done by fluoroscopy, ultrasound or by other location methods such as magnetizing one of a set of opposing elongated arms 6a. This creates a north and south pole 15, as illustrated in Figures 3-A and 3-B, which can be detected from outside the body by a magnetometer or sensitive magnetic compass.

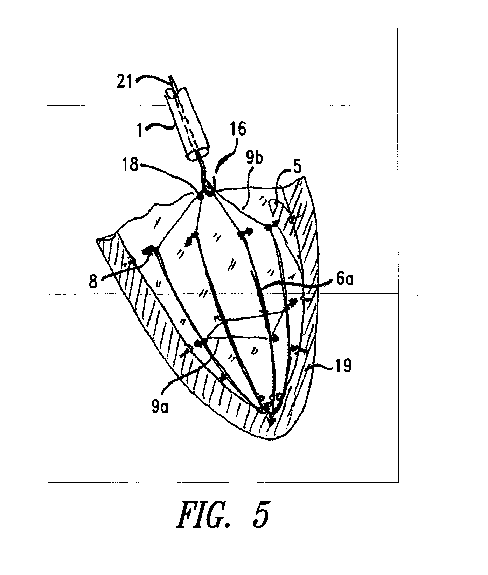

In some embodiments, device 4 allows for aborting installation at any stage and retrieving the device 4. As illustrated in Figure 5, a flexible cable 21 terminating in a hook 16 may be introduced into left ventricle 2 to retrieve device 4 by snagging restraining members 9 with hook 16 and pulling device 4 back into catheter assembly 1. Restraining members 9 are joined at a crossover point 18. This allows the hook 16 to self-center without regard to the precise the location at which the hook 16 snagged the restraining members 9 and without regard to whether the hook 16 snagged one or more of the restraining members 9. Retrieval is more difficult once scar tissue has developed.

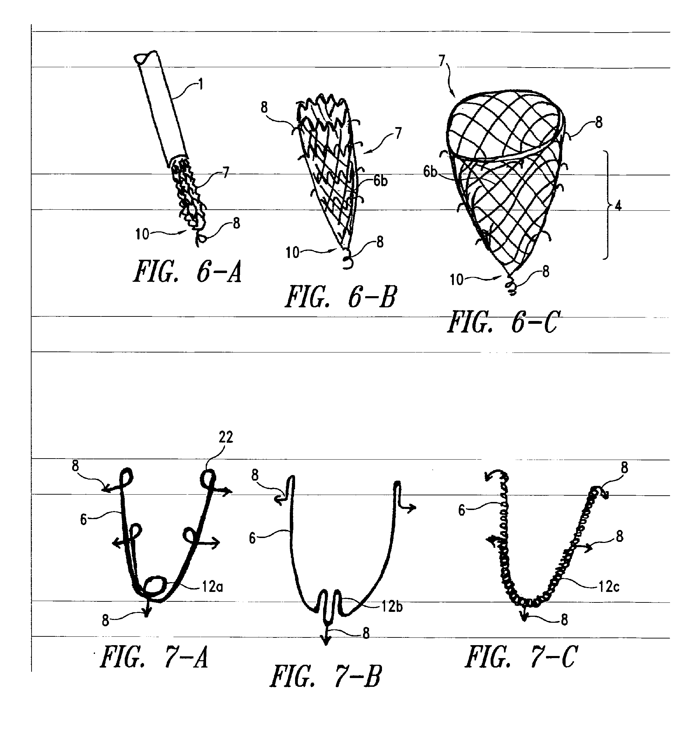

Figures 6-A, 6-B and 6-C show examples not forming part the invention of a medical device for percutaneous implantation in various stages of deployment. The device 4 has a plurality of elastically deformable elements 6b that form an elongated tubular structure 7 having an apex 10 at one end and optional barbs 8. The tubular structure 7 is elastically and compressably deformable from a relatively radially expanded conical configuration, as shown in Figure 6-C, to a relatively radially unexpanded cylindrical configuration, as shown in Figure 6-B. When in the relatively radially unexpanded cylindrical configuration, the tubular structure 7 is capable of being retained in a catheter assembly 1, as illustrated in Figure 6-A. The catheter size is preferably in the same range as catheters used in other percutaneous cardiac procedures.

The elongated mesh tubular structure 7 is formed by methods such as, for example, weaving, braiding or knitting, resulting in a plurality of intersections of mesh elements. In some embodiments, at least some of the intersections of mesh elements are coupled or fixed together, for example, wire welded structures. The elongated mesh tubular structure 7 tapers from a distal end to an apex 10. In some embodiments, a common connector member (not shown) is located at the apex 10 to facilitate implantation of the device 4. Elements 6b that form elongated mesh tubular structure 7 can be made from any durable, elastically deformable material, for example, Nitinol, spring tempered stainless steel, plated beryllium copper or polymeric material.

With reference to Figures 7-A, 7-B and 7-C, in various examples not forming part of the invention, a pair of generally opposed elastically deformable arms 6 include at least one bend 12 to increase the elastic range of such arms 6. When present, bends 12 may enhance the performance of the device by allowing the use of a smaller device without sacrificing the ability to assist diastolic function. This is achieved by distributing elastic deformation over a greater length of the arms 6. When bends 12 are not present a relatively larger catheter is required to implant the device because attempting to compress the device in a volume similar to that which is possible with bends 12 would result in plastic deformation of the elastically deformable arms 6, as even the most elastic metals, for example, Nitinol, have a minimum bend radius before deforming plastically.

By way of example, by adding bends 12 in the form of small loops 12a, preferably in the range of about 1 to 5mm in diameter, a significantly larger elastic range of elastically deformable elements 6 in the form of elongated arms 6a is achieved. For example, when using 0.5mm Nitinol wire the smallest catheter needed to accommodate elongated arms 6a without a loop 12a (and still have elastic range left to perform diastolic assist) was 8mm while the smallest catheter need to accommodate elongated arms 6a with a loop 12a was 3mm. It is appreciated that similar performance gains can be achieved using bends 12 in various other configurations, for example, V-shape bends 12b or a continuous spiral 12c.

Bends 12 also increase the fatigue life of the device. Over the life of a patient, a device may have to flex over a billion times. The phenomenon known as metal fatigue sets a limit to the fatigue life of the device. It is well known in the art of engineering that in order to achieve a long fatigue life the device has to flex a small portion of its elastic range, typically about 10%-40%. Therefore, it is preferable that the operating flexing range should be a small portion of the total elastic range. For any desired flexing range, an increase in the elastic range will necessarily reduce the proportion of flex range to elastic range and thus increase the fatigue life of the device.

As illustrated in Figures 7-A, 7-B and 7-C, bends 12 may take the form of various shapes including a complete loop 12a, a V-shape 12b, or a continuous spiral 12c. However, it is appreciated that bends 12 make take the form of numerous other configurations that allow elastic deformation to be distributed over a greater length of the elastically deformable elements 6. In some embodiments, more than one bend 12 can be used in order to further increase the elastic range. In other embodiments, bends 12 may have one or more turns in order to optimize the elastic range of the elastically deformable elements 6. In a similar manner, bends 22, similar to bends 12, can be formed at the base of optional barbs 8. Such bends allow the barbs 8 to be folded into a smaller diameter catheter.

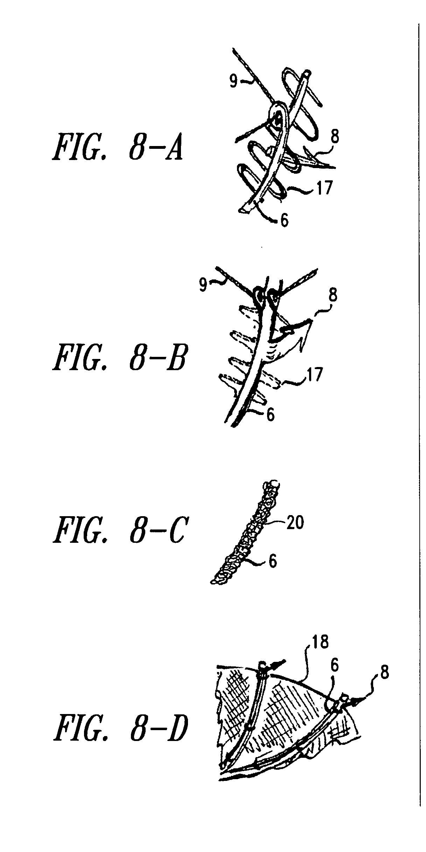

Figure 8-A provides, according to one illustrated example not forming part of the invention, a partial view of elastically deformable element 6 of a medical device 4 comprising a barb 8, a load spreading structure 17, and restraining member 9. In the illustrated embodiment, elastically deformable elements 6 are made of spring wire, restraining members 9 are made of thin stainless steel cable, and barbs 8 are made of steel wire spot welded to elastically deformable elements 6. The load spreading structure 17 comprises bent wire that is spot welded to elastically deformable elements 6 as shown. Alternatively, load spreading structure 17 may be made of a polymeric strip. In some embodiments, device 4 can be coated with an anti-coagulant coating, drug eluting coating or any beneficial coating well known from the art of stents.

Figure 8-B provides, according to another illustrated example not forming part of the invention, a partial view of elastically deformable elements 6 of a medical device 4 comprising a barb 8 and an optional load spreading structure 17 cut out from a single sheet of elastic material and bent to shape as shown. This embodiment is particularly advantageous when device 4 is made of Nitinol, as Nitinol is difficult to join.

Figure 8-C provides, according to another illustrated example not forming part of the invention, a partial view of elastically deformable elements 6 of a medical device 4 comprising a surface finish or texture 20 (as opposed to discrete barbs) to promote bonding with a ventricular wall. Examples of surface finish 20 include: porous surfaces, surfaces coated with biological adhesives, surfaces coated, covered or studded with miniature barbs similar to well known hook and loop fasteners, and growth-promoting drug coatings. For example, velour-like finishes promote tissue infiltration and greatly increase bonding strength. Test results are listed in US Patent No. 4,164,046 example not forming part of the invention.

Figure 8-D provides, according to another illustrated example not forming part of the invention, a partial view of elastically deformable elements 6 of a medical device 4 comprising barbs 8 and a continuous layer of a flexible mesh or flexible hemostatic material 18, such as Dacron fabric. In examples wherein layer 18 is hemostatic the device 4 can seal an aneurysm or puncture in the ventricular wall 19. This may be particularly desirable when the ventricular wall is already significantly thinned by enlargement.

## Claims

1. A percutaneously implantable diastolic assist medical device, comprising:
at least four elongated arms (6a) that extend from an apex (10) and have a respective distal end spaced from the apex (10), the elongated arms (6a) compressably elastically deformable from a relatively radially expanded configuration to a relatively radially unexpanded configuration, where the respective distal end of each of the elongated arms is spaced relatively away from one another in the relatively radially expanded configuration and the respective distal end of each of the elongated arms is spaced relatively close to one another in the relatively radially unexpanded configuration, wherein each of the elongated arms (6a) has at least one bend (12) therein such that the elongated arm is bent back on itself, the elongated arms (6a) elastically biased toward the relatively radially expanded configuration while in the relatively unexpanded configuration, the at least one bend (12) increasing an elastic range of the elongated arm; and a number of restraining members (9), , wherein the restraining members (9b) limit a radial expansion of the elongated arms (6a) relative to one another, **characterized in that** at least one of the restraining members (9b) extends between each pair of a number of pairs of opposing ones of the elongated arms (6a).

2. The medical device of claim 1 wherein each of the elongated arms (6a) has a first bend (12) proximate the apex (10) and a second bend (12) proximate the distal end of the elongated arm.

3. The medical device of claim 2 wherein there is at least a third bend (12) between the first and the second bends.

4. The medical device of any of claims 1 through 3 wherein the at least one bend (12) in each of the elongated arms (6a) forms a complete loop (12a).

5. The medical device of any of claims 1 through 4 wherein each pair of opposed ones of the elongated arms (6a) are a unitary elastic member.

6. The medical device of any of claims 1 through 4 wherein each pair of opposed ones of the elongated arms (6a) are an integral member.

7. The medical device of any of claims 1 through 4 wherein each of the elongated arms (6a) is a distinct member physically coupled to a common connector member (14) wherein the common connector member (14) comprises a circular ring (14a) wherein the plurality of elongated arms (6a) are connected to the periphery of the circular ring (14a) and spaced at substantially equal intervals.

8. The medical device of claim 7, further comprising:
at least one barb (8) located at the common connector member (14) and oriented outwardly from the medical device (4)

9. The medical device of any of claims 1 through 4 wherein each of the elongated arms (6a) is a distinct member physically coupled to a common connector member (14) wherein the common connector member (14) comprises a threaded member (14b), the threaded member (14b) configured to engage corresponding threads (13) of a catheter assembly (1).

10. The medical device of claim 7, further comprising:
a catheter assembly (1) capable of retaining the elongated arms (6a) in the relatively radially unexpanded configuration within a cavity of the catheter assembly (1) and having delivery means adapted to engage the common connector member (14).

11. The medical device of any of claims 1 through 4 wherein the elongated arms (6a) comprise a shape memory material.

12. The medical device of any of claims 1 through 4 wherein the elongated arms (6a) comprise Nitinol.

13. The medical device of any of claims 1 through 4 wherein at least some of the elongated arms (6a) have at least one barb (8) at least proximate the distal end thereof.

14. The medical device of any of claims 1 through 4 wherein at least some of the elongated arms (6a) have a rough surface finish (20) at least proximate the distal end thereof.

15. The medical device of any of claims 1 through 4 wherein at least some of the elongated arms (6a) have a load spreading structure (17) at least proximate the distal end thereof.

16. The medical device of any of claims 1 through 4 wherein the medical device in the radially unexpanded configuration is capable of retention in a catheter assembly (1).

17. The medical device of any of claims 1 through 4 wherein the elongated arms (6a) form a cone-like shape when in the expanded configuration.

## Patentansprüche

1. Eine perkutan implantierbare medizinische Diastolen-Unterstützungsvorrichtung, aufweisend:
mindestens vier langgestreckte Arme (6a), die sich von einem Scheitelpunkt (10) aus erstrecken und ein jeweiliges distales Ende aufweisen, das im Abstand von dem Scheitelpunkt (10) angeordnet ist, wobei die langgestreckten Arme (6a) aus einer relativ radial expandierten Konfiguration in eine relativ radial nicht-expandierte Konfiguration zusammendrückbar elastisch verformbar sind, wobei das jeweilige distale Ende von jedem der langgestreckten Arme in der relativ radial expandierten Konfiguration relativ voneinander entfernt angeordnet ist und das jeweilige distale Ende von jedem der langgestreckten Arme in der relativ radialen nicht-expandierten Konfiguration relativ nahe beieinander angeordnet ist, wobei jeder der langgestreckten Arme (6a) mindestens eine Krümmung (12) darin aufweist, derart, dass der langgestreckte Arm um sich selbst umgebogen ist, wobei die langgestreckten Arme (6a) in Richtung zu der relativ radial expandierten Konfiguration elastisch vorgespannt sind, während sie in der relativ nicht-expandierten Konfiguration sind, wobei die mindestens eine Krümmung (12) einen elastischen Bereich des langgestreckten Arms vergrößert, und eine Anzahl von Beschränkungselementen (9), wobei die Beschränkungselemente (9b) eine radiale Ausdehnung der langgestreckten Arme (6a) bezüglich einander beschränken, **dadurch gekennzeichnet, dass** mindestens eines der Beschränkungselemente (9b) sich zwischen jedem Paar einer Anzahl von Paaren von einander gegenüberliegenden der langgestreckten Arme (6a) erstreckt.

2. Die medizinische Vorrichtung gemäß Anspruch 1, wobei jeder der langgestreckten Arme (6a) eine erste Krümmung (12) nahe bei dem Scheitelpunkt (10) und eine zweite Krümmung (12) nahe bei dem distalen Ende des langgestreckten Arms aufweist.

3. Die medizinische Vorrichtung gemäß Anspruch 2, wobei mindestens eine dritte Krümmung (12) zwischen der ersten und der zweiten Krümmung vorgesehen ist.

4. Die medizinische Vorrichtung gemäß irgendeinem der Ansprüche 1 bis 3, wobei die mindestens eine Krümmung (12) in jedem der langgestreckten Arme (6a) eine vollständige Schleife (12a) bildet.

5. Die medizinische Vorrichtung gemäß irgendeinem der Ansprüche 1 bis 4, wobei jedes Paar von einander gegenüberliegenden der langgestreckten Arme (6a) ein einheitliches elastisches Element ist.

6. Die medizinische Vorrichtung gemäß irgendeinem der Ansprüche 1 bis 4, wobei jedes Paar von einander gegenüberliegenden der langgestreckten Arme (6a) ein integrales Element ist.

7. Die medizinische Vorrichtung gemäß irgendeinem der Ansprüche 1 bis 4, wobei jeder der langgestreckten Arme (6a) ein eigenes Element ist, das physikalisch mit einem gemeinsamen Verbindungselement (14) verbunden ist, wobei das gemeinsame Verbindungselement (14) einen kreisförmigen Ring (14a) aufweist, wobei die Mehrzahl von langgestreckten Armen (6a) mit dem Umfang des kreisförmigen Rings (14a) verbunden ist und in im Wesentlichen gleichen Abständen angeordnet ist.

8. Die medizinische Vorrichtung gemäß Anspruch 7, ferner aufweisend:
mindestens einen Stachel (8), der an dem gemeinsamen Verbindungselement (14) angeordnet ist und von der medizinischen Vorrichtung (4) aus in Richtung nach außen ausgerichtet ist.

9. Die medizinische Vorrichtung gemäß irgendeinem der Ansprüche 1 bis 4, wobei jeder der langgestreckten Arme (6a) ein eigenes Element ist, das physikalisch mit einem gemeinsamen Verbindungselement (14) verbunden ist, wobei das gemeinsame Verbindungselement (14) ein Gewindeelement (14b) aufweist, wobei das Gewindeelement (14b) eingerichtet ist, um mit korrespondierenden Gewinden (13) einer Kathetervorrichtung (1) in Eingriff zu sein.

10. Die medizinische Vorrichtung gemäß Anspruch 7, ferner aufweisend:
eine Kathetervorrichtung (1), die in der Lage ist, die langgestreckten Arme (6a) in der relativ radial nicht-expandierten Konfiguration innerhalb eines Hohlraums der Kathetervorrichtung (1) zu halten, und die Abgabemittel aufweist, die für einen Eingriff mit dem gemeinsamen Verbindungselement (14) angepasst sind.

11. Die medizinische Vorrichtung gemäß irgendeinem der Ansprüche 1 bis 4, wobei die langgestreckten Arme (6a) ein Formgedächtnismaterial aufweisen.

12. Die medizinische Vorrichtung gemäß irgendeinem der Ansprüche 1 bis 4, wobei die langgestreckten Arme (6a) Nitinol aufweisen.

13. Die medizinische Vorrichtung gemäß irgendeinem der Ansprüche 1 bis 4, wobei zumindest einige der langgestreckten Arme (6a) mindestens einen Stachel (8) zumindest in der Nähe des distalen Endes davon aufweisen.

14. Die medizinische Vorrichtung gemäß irgendeinem der Ansprüche 1 bis 4, wobei zumindest einige der langgestreckten Arme (6a) zumindest in der Nähe des distalen Endes davon eine raue Oberflächenbeschaffenheit (20) aufweisen.

15. Die medizinische Vorrichtung gemäß irgendeinem der Ansprüche 1 bis 4, wobei zumindest einige der langgestreckten Arme (6a) zumindest in der Nähe des distalen Endes davon eine Lastverteilungsstruktur (17) aufweisen.

16. Die medizinische Vorrichtung gemäß irgendeinem der Ansprüche 1 bis 4, wobei die medizinische Vorrichtung in der radial nicht-expandierten Konfiguration zum Halten in einer Kathetervorrichtung (1) in der Lage ist.

17. Die medizinische Vorrichtung gemäß irgendeinem der Ansprüche 1 bis 4, wobei die langgestreckten Arme (6a) eine kegelartige Form ausbilden, wenn sie in der expandierten Konfiguration sind.

## Revendications

1. Dispositif médical assisté diastolique implantable par voie percutanée, comprenant :
au moins quatre bras allongés (6a) qui s'étendent depuis un sommet (10) et ont une extrémité distale respective espacée du sommet (10), les bras allongés (6a) étant élastiquement déformables de manière compressible d'une configuration relativement radialement étendue à une configuration relativement radialement non étendue, où les extrémités distales respectives de chacun des bras allongés sont espacées relativement loin les unes par rapport aux autres dans la configuration relativement radialement étendue et les extrémités distales respectives de chacun des bras allongés sont espacées relativement près les unes des autres dans la configuration relativement radialement non étendue, dans lequel chacun des bras allongés (6a) a au moins une pliure (12) à l'intérieur, de sorte que le bras allongé soit replié sur lui-même, les bras allongés (6a) étant élastiquement inclinés vers la configuration relativement radialement étendue tandis que dans la configuration relativement non étendue, ladite au moins une pliure (12) augmente un domaine d'élasticité du bras allongé ; et un certain nombre d'éléments de retenue (9), dans lesquels les éléments de retenue (9b) limitent une expansion radiale des bras allongés (6a) les uns par rapport aux autres, **caractérisé en ce qu'**au moins un des éléments de retenue (9b) s'étend entre chaque paire d'un certain nombre de paires opposées de bras allongés (6a)

2. Dispositif médical selon la revendication 1, dans lequel chacun des bras allongés (6a) a une première pliure (12) proche du sommet (10) et une seconde pliure (12) proche de l'extrémité distale du bras allongé.

3. Dispositif médical selon la revendication 2, dans lequel il existe au moins une troisième pliure (12) entre la première et la seconde pliures.

4. Dispositif médical selon l'une quelconque des revendications 1 à 3, dans lequel ladite au moins une pliure (12) dans chacun des bras allongés (6a) forme une boucle complète (12a).

5. Dispositif médical selon l'une quelconque des revendications 1 à 4, dans lequel chaque paire opposée de bras allongés (6a) est un élément élastique unitaire.

6. Dispositif médical selon l'une quelconque des revendications 1 à 4, dans lequel chaque paire opposée de bras allongés (6a) est un élément d'un seul tenant.

7. Dispositif médical selon l'une quelconque des revendications 1 à 4, dans lequel chacun des bras allongés (6a) est un élément distinct physiquement raccordé à un élément de connecteur commun (14), dans lequel l'élément de connecteur commun (14) comprend un anneau circulaire (14a), dans lequel la pluralité de bras allongés (6a) est connectée à la périphérie de l'anneau circulaire (14a) et espacée à intervalles sensiblement égaux.

8. Dispositif médical selon la revendication 7, comprenant en outre :
au moins un picot (8) situé au niveau de l'élément de connecteur commun (14) et orienté vers l'extérieur par rapport au dispositif médical (4).

9. Dispositif médical selon l'une quelconque des revendications 1 à 4, dans lequel chacun des bras allongés (6a) est un élément distinct, physiquement raccordé à un élément de connecteur commun (14), dans lequel l'élément de connecteur commun (14) comprend un élément fileté (14b), l'élément fileté (14b) étant configuré pour mettre en prise des filets correspondants (13) d'un ensemble de cathéter (1).

10. Dispositif médical selon la revendication 7, comprenant en outre :
un ensemble de cathéter (1) capable de retenir les bras allongés (6a) dans la configuration relativement radialement non étendue dans une cavité de l'ensemble de cathéter (1) et ayant des moyens de distribution adaptés pour mettre en prise l'élément de connecteur commun (14).

11. Dispositif médical selon l'une quelconque des revendications 1 à 4, dans lequel les bras allongés (6a) comprennent un matériau à mémoire de forme.

12. Dispositif médical selon l'une quelconque des revendications 1 à 4, dans lequel les bras allongés (6a) comprennent du Nitinol.

13. Dispositif médical selon l'une quelconque des revendications 1 à 4, dans lequel au moins certains des bras allongés (6a) ont au moins un picot (8) au moins près de leur extrémité distale.

14. Dispositif médical selon l'une quelconque des revendications 1 à 4, dans lequel au moins certains des bras allongés (6a) ont une finition de surface rugueuse (20) au moins près de leur extrémité distale.

15. Dispositif médical selon l'une quelconque des revendications 1 à 4, dans lequel au moins certains des bras allongés (6a) ont une structure de répartition de charge (17) au moins près de leur extrémité distale.

16. Dispositif médical selon l'une quelconque des revendications 1 à 4, dans lequel le dispositif médical dans la configuration radialement non étendue est capable de rétention dans un ensemble de cathéter (1).

17. Dispositif médical selon l'une quelconque des revendications 1 à 4, dans lequel les bras allongés (6a) forment un cône quand ils sont dans la configuration étendue.
